# EUROPEAN PATENT APPLICATION

(11) **EP 1 559 684 A1**
(43) Date of publication of application: **03.08.2005**
(21) Application number: 05250573.2
(22) Date of filing: 02.02.2005
(51) Int. Cl.: C02F 1/28, B01J 20/24

(54) **Method of separating a substance from a liquid**

(30) Priority: 02.02.2004 JP 2004026081
(71) Applicant: CANON KABUSHIKI KAISHA, Ohta-ku, Tokyo (JP)
(72) Inventor: Zhang, Zuyi, Ohta-ku Tokyo (JP); Sakakibara, Teigo, Ohta-ku Tokyo (JP); Kotani, Yoshinori, Ohta-ku Tokyo (JP); Yuasa, Toshiya, Ohta-ku Tokyo (JP); Nishi, Norio, Sapporo-shi Hokkaido (JP)
(74) Representative: Beresford, Keith Denis Lewis

(57) **Abstract**

A liquid containing a separation target substance is brought into contact with a double helix DNA-holding phase to have the separation target substance held by the double helix DNA-holding phase, which is then removed from the liquid.

The contact of the double helix DNA with the separation target substance is performed in a liquid medium having a salt concentration of 0.02 mass% or more relative to the total mass of the liquid phase.

## Description

### BACKGROUND OF THE INVENTION

### Field of the Invention

The present invention relates to a system for separation treatment of a substance from a liquid by using a double helix DNA, and more particularly to a treating system for separating a separation target substance from a treatment target liquid involving selectively adsorbing the separation target substance on the DNA under conditions in which a DNA structure is more stable. The present invention further relates to a method of treating a liquid using this treating system.

### Related Background Art

Conventionally, it has been known that a DNA forms a double helix structure and bears genetic information in a living organism. An aromatic compound, which has a planar chemical structure, is apt to be selectively intercalated into the double helix of a DNA, and this intercalation sometimes causes the DNA itself to vary, thereby giving rise to carcinogenicity. Making the best of this peculiar nature of the DNA, a method of selectively removing a toxic substance such as a carcinogenic compound by means of a double helix DNA has been proposed (Function & Materials, Vol. 19, 1999).

To use the double helix DNA as an environmental purification material, technology for immobilizing the double helix DNA has been investigated. Japanese Patent Application Laid-Open No. H07-041494 discloses a method of immobilizing deoxyribonucleic acids involving coagulating an alkali metal salt of a deoxyribonucleic acid and an alkali metal salt of an alginic acid with a divalent metal-containing compound. Japanese Patent Application Laid-Open No. 2001-081098 discloses immobilization of a DNA on a support by irradiating an aqueous solution of DNA or liquid film of a DNA on the support, or a thin layer of a water-soluble DNA on the support with ultraviolet rays having a wavelength of 250 to 270 nm for hardening the DNA where the immobilized DNA is used as an environmental purification material. Japanese Patent Application Laid-Open No. H10-175994 discloses a DNA-immobilized composite in which a carrier of an inorganic solid is used. Further, development of a porous DNA-carried product has been attempted from the viewpoint of effective utilization of inside DNA in a carried product and improvement in purification speed. Further, an aqueous DNA solution is brought into contact with an aqueous solution containing dioxins through a hollow dialysis membrane, and it has been confirmed that the dioxins permeate through the dialysis membrane and are adsorbed on the DNA (High Polymers, Vol. 52, 2003, p134-137).

Japanese Patent Application Laid-Open No. 2002-218976 discloses plasma treatment of a substrate with atomic oxygen plasma before a nucleic acid is immobilized on the substrate.

### SUMMARY OF THE INVENTION

Each of the above-mentioned documents does not mention how a double helix structure of a DNA is retained. When a DNA is brought into contact with water, fluctuation tends to occur in the double helix structure of the DNA, resulting in unfolding of the double helix structure. Such a phenomenon is considered to occur more often, particularly at elevated temperature. When a DNA is carried on an inorganic carrier, a problem of how to develop a mechanical strength of a matrix (base member that constitutes the carrier) remains to be solved.

As described above, when a DNA material is applied to a water purification system based on intercalation characteristics of the DNA, a method of maintaining the function of DNA under a wide range of conditions is demanded. The present invention has been made in order to solve such problems in the technology and is aimed at providing a system for treating a liquid that allows stable development of an adsorbing ability of a double helix and that can be used advantageously for purification of water or the like.

The present invention relates to a system for treating a liquid containing a separation target substance including: a treating region having arranged therein a double helix DNA-holding phase; liquid supply means for supplying a liquid containing the separation target substance to the treating region; and recover means for recovering at least one of 1) and 2) below, 1) the DNA-holding phase in which the separation target substance is held or concentrated through contact with the double helix DNA-holding phase, and 2) a liquid in which the separation target substance is removed or a concentration of the separation target substance is decreased, in which a concentration of a salt compound in a liquid phase where the contact of the double helix DNA with the separation target substance occurs is 0.02 mass% or more.

Further, the present invention relates to a separation treatment method for separating a separation target substance from a liquid using the treating system having the above-mentioned constitution, the method including the steps of: preparing a liquid containing a separation target substance and having a salt compound concentration of 0.02 mass% or more; supplying the liquid to a treating region and bringing the liquid into contact with a phase that holds a double helix DNA to have the separation target substance held by the DNA-holding phase; recovering the DNA-holding phase in which the separation target substance is held or concentrated through contact of the liquid with the double helix DNA-holding phase, and/or the liquid from which the separation target substance is removed or reduced to a lower concentration.

The present invention allows stable development of the function of removing a separation target substance from a liquid in a treating system by use of an aqueous DNA solution, a DNA dispersion, or a solid phase having a DNA in the presence of a salt compound. In the case of an inorganic DNA-carrying product, the mechanical properties thereof are retained. Thus, the present invention provides an environmental purification system that allows development of a purification function in a wide range of environmental conditions.

Other features and advantages of the present invention will be apparent from the following description.

### DETAILED DESCRIPTION OF THE PREFERRED EMBODIMENTS

Preferred embodiments of the present invention will now be described in detail.

The present invention provides a system for performing separation of a separation target substance from a liquid phase in the presence of a salt compound, in which the separation target substance selectively migrates into a phase containing a double helix DNA and is held and concentrated therein.

Here, the term "separation target substance" refers to a substance that interacts with and is held by a double helix DNA through intercalation or adsorption. Examples of the separation target substance include toxic substances that adversely influence a structure of the DNA or adversely influence genetic information of the DNA due to such interaction as mentioned above. The substances that can interact with the double helix DNA have not been fully clarified, but examples thereof include substances each having an aromatic functional group that is intercalated into a DNA, and heavy metal ions that are selectively adsorbed on a DNA. Specific examples thereof include: dioxins such as polychlorodibenzo-para-dioxin, polychlorodibenzofuran, and polychlorobiphenyl (PCB); benzo[a]pyrene; dichlorodiphenyltrichloroethane (DDT); diethylstilbestrol (DES); ethidium bromide; acridine orange; and derivatives thereof. For example, dioxins and the like are generated through waste incineration. A part of thus generated toxic substances is selectively intercalated into the DNA and serves as a separation target substance in the separation system of the present invention. When water contaminated with agricultural chemicals such as PCB is purified, the toxic substances that adversely influence the DNA also collectively serve as a separation target substance in the present invention. Further, a trace amount of a carcinogenic substance that influences the DNA in synthetic drugs serves as the separation target substance.

In the present invention, a holding phase that can hold a double helix DNA in spite of a liquid to be treated, that is, that can hold the double helix DNA in a state where the double helix DNA will not migrate into the liquid when the double helix DNA is brought into contact with the liquid to be treated is used.

Examples of the holding phase that can be used include a liquid phase in which a double helix DNA is dissolved or dispersed in a liquid, and a solid phase in which a double helix DNA is carried or held on a solid matrix. When the liquid phase is used, a constitution, in which the liquid phase and a liquid to be treated are arranged such that the liquid phase and the liquid are brought into contact with each other through an appropriate permeable membrane such as a semipermeable membrane, can be used.

The liquid containing a separation target substance is brought into contact with the double helix DNA-holding phase in an aqueous medium having a salt compound concentration of 0.02 mass% or more. When the holding phase is a liquid phase, the aqueous medium forms the liquid phase, which can be brought into contact with a liquid containing a separation target substance having a salt compound concentration of 0.02 mass% or more, or to which a salt compound is added to the aqueous medium, so that the salt compound coricentration of the aqueous medium reaches 0.02 mass% or more. For example, when the liquid containing the separation target substance is an aqueous solution of the separation target substance, the salt compound concentration of the aqueous solution is adjusted to 0.02 mass% or more in a stage where the aqueous solution and the aqueous medium containing the double helix DNA that is brought into contact with the aqueous solution reach equilibrium with respect to the concentration of the salt compound. When the double helix DNA-holding phase is a solid phase, the medium of the liquid itself that is brought into contact with the solid phase serves as an aqueous medium, and the salt compound concentration of the aqueous medium is adjusted to 0.02 mass% or more. A liquid to be treated having a salt compound concentration of 0.02 mass% or more can be used as it is. The salt compound concentration of the liquid to be treated can be obtained by directly measuring ion species and calculating amounts thereof respectively by means of ion chromatography analysis, atomic absorption analysis, and the like methods. Also, a method that can be used involves: bringing the liquid to be treated to equilibrium with deionized water or pure water sufficiently through a dialysis membrane; concentrating the obtained aqueous solution; and measuring a salt content thereof.

The salt compound for maintaining or adjusting the salt compound concentration of the aqueous phase, in which the separation target substance and the double helix DNA are brought into contact with each other, is used for the purpose of stabilizing the double helix structure of the DNA.

The term "salt compound" as used herein means a compound formed from an acid and a base. Suitable salt compounds include hydrated salt compounds, and salt compounds of alkali metals and alkaline earth metals are especially preferred. Salt compounds of transition metals may also be used.

The salt compound is not particularly limited so far as it is water-soluble and has the desired stabilizing effect. Specific examples of the salt compound include: alkali metal salts such as sodium chloride, sodium nitrate, sodium sulfate, sodium phosphate, sodium carbonate, sodium acetate, sodium formate, potassium chloride, potassium nitrate, potassium sulfate, potassium carbonate, potassium acetate, potassium phosphate, lithium chloride, lithium nitrate, lithium sulfate, and lithium phosphate; alkaline earth metal salts such as magnesium chloride, magnesium nitrate, calcium chloride, calcium carbonate, calcium nitrate, barium chloride, and barium nitrate; aluminum chloride; and aluminum nitrate. The salt compound may be used singly or two or more kinds of salts may be used simultaneously. Examples of a particularly preferable salt include sodium chloride, potassium chloride, magnesium chloride, and calcium chloride. It is preferable that an aqueous solution contains at least one of the salt compounds.

The concentration of the salt compound is 0.02% (mass) or more, preferably 0.1% or more. When the salt concentration is 0.02% or more, the double helix structure of the DNA is maintained under a wide range of conditions and a separation function is developed. The upper limit of the concentration of the salt compound is not particularly limited in the case of a circulating system in which no salt compound is discharged in the form of wastewater. When the salt compound is discharged in the form of wastewater, the upper limit of the concentration of the salt compound is set to preferably 10% or less and more preferably 5% or less.

An already existing salt compound may be used as the salt compound in the liquid containing the separation target substance as described previously, but when the salt compound does not have a desired concentration, a salt compound is added to the system to adjust the concentration of the salt compound. Also, a technique that may be used involves integrating a necessary amount of a salt compound with an aqueous DNA solution or a solid phase having carried thereon a DNA, and allowing the salt compound to be dissolved in the system, to thereby increase the concentration of the salt compound to a desired level.

Regarding the DNA that can be used in the present invention, any DNA may be used for the purpose of the present invention so far as the DNA is of a double helix that has an intercalation function. For example, a DNA obtained from testis of mammals or thymus of animals can be used. In particular, a DNA obtained from milt (testis) of a salmon, a herring, or a cod is preferable. Further, a DNA obtained from thymus of mammals or birds such as cows, pigs, and chickens is preferable. Examples of other water-soluble DNAs include synthetic DNAs, particularly a DNA sequence having a (dA)-(dT) base pair such as a DNA that has a sequence of a type poly(dA)-poly(dT). The DNA is used in a form of an alkali salt or ammonium salt in a water-soluble form. A molecular weight of the DNA is preferably 100,000 or more, more preferably 500,000 or more.

When the aqueous DNA solution is used as a holding phase, the aqueous solution of the DNA and water to be treated containing a separation target substance (for example, contaminated water) are brought into contact with each other through a separating membrane. The separating membrane is not particularly limited so far as it does not allow permeation of DNA polymers but allows passing of the separation target substance. A molecular weight cutoff of the separating membrane is 500 or more, preferably 1,000 or more. If the molecular weight cutoff is 500 or more, toxic substances each having a low molecular weight can easily pass through the separating membrane while the passing of DNA molecules is prevented. Examples of materials of the separating membrane include: dialysis membranes made of cellulose ester, regenerated cellulose, and polyvinylidene; composite cellulose hollow filters; polyester sulfone hollow filters; and polysulfone hollow filters. To increase an adsorption speed, the aqueous solution of the DNA, the water containing the separation target substance, or both may be circulated or passed.

The concentration (by mass) of the DNA in the aqueous solution is preferably 0.005% to 10%, more preferably 0.1% to 5%. If the concentration of the DNA is 0.005% or more, efficient purification is possible. On the other hand, if the concentration of the DNA is 10% or less, a viscosity of the aqueous DNA solution becomes appropriate, allowing the aqueous DNA solution to flow.

In the present invention, the double helix structure of the DNA is stabilized, and thus, the separation function is also developed in a temperature region higher than a temperature region of a conventional separation system.

When a solid phase in which DNA has been carried thereon and insolubilized is used, the solid phase is brought into direct contact with a liquid containing a separation target substance. The solid phase having the DNA carried thereon is not particularly limited, and any solid phase may be used that contains the DNA without hindering the function thereof and which is insoluble in the liquid as a treatment target. Examples of the solid phase include an insolubilized DNA, a DNA held on an inorganic matrix and insolubilized, and a DNA held on an organic matrix and insolubilized.

The insolubilized DNA can be obtained by crosslinking or modifying a DNA. Some following methods may be used. However, the present invention should not be considered to be limited to these methods.

For example, the DNA can be insolubilized with a metal ion that crosslinks with a phosphate group of the DNA. Specific examples of the metal ion include Mg²⁺, Ca²⁺, Ba²⁺, Sr²⁺, Al³⁺, Fe³⁺, Ti⁴⁺, and Zr⁴⁺. An example of the insolubilization method that can also be used is a method involving appropriately reacting an aqueous solution of a metal compound and an aqueous DNA solution to obtain a water-insoluble gel form of the DNA. The amount of the metal ion or metal compound based on the amount of DNA is 0.01% to 5%, preferably 0.05% to 2% in terms of mass of metal oxide.

The DNA can be insolubilized with excited rays. For example, an aqueous DNA solution may be applied onto a base member and dried, and then the DNA may be hardened with ultraviolet rays, X rays, y rays, or electron beam. The conditions of hardening are not particularly limited. When the DNA is partially insolubilized, a soluble DNA may be extracted with water or the like to use only an insolubilized DNA. When ultraviolet rays are used, the wavelength of the ultraviolet rays is preferably 400 nm or less, more preferably 350 nm or less. When electron beam is used, an acceleration voltage thereof is 1 kV or more, preferably 5 kV or more.

Also, the phosphate group of a DNA can be lipidated to insolubilize the DNA. For example, a water-insoluble DNA can be obtained by reacting an aqueous alkali solution of a DNA with a quaternary ammonium salt. Specific examples of the quaternary ammonium salt that can be used include n-hexadodecyltrimethylammonium chloride, benzyldimethylhexadecylammonium chloride, and didodecyldimethylammonium bromide.

When a DNA is immobilized by using an inorganic matrix (base member), particularly an oxide matrix, oxide fine particles, metal ion salts, a metal alkoxide, and so on are used as raw materials for oxides. An oxide colloid and a metal alkoxide are preferably used.

When an oxide colloid is used, a particle size of the colloid is preferably 5 nm to 100 nm, more preferably 10 nm to 50 nm. If the particle size of the colloid is larger than 5 nm, a pore size of the matrix will not be too small and DNA polymers can be advantageously carried thereon. On the other hand, if the particle size of the colloid is smaller than 100 nm, the number of pores will not decrease. Examples of the oxide particles include colloidal silica, colloidal aluminum oxide, colloidal iron oxide, colloidal gallium oxide, colloidal lanthanum oxide, colloidal titanium oxide, colloidal cerium oxide, colloidal zirconium oxide, colloidal tin oxide, and colloidal hafnium oxide. The oxide particles may be used singly or two or more kinds of them may be used in combination. From the viewpoint of economical efficiency, it is preferable that an easily available silica colloid be used as a main component of the matrix. Specific examples of the colloidal silica include: water-based sol brands such as SNOWTEX 20, SNOWTEX 30, SNOWTEX N, SNOWTEX O, and SNOWTEX C, methanol-based sols, solvent-based sol brands such as IPA-ST, EG-ST, and MEK-ST, all available from Nissan Chemical Industries, Ltd.; and solvent-based sol brands such as OSCAL-1132, OSCAL-1432, and OSCAL-1232 available from Catalysts & Chemicals Industries Co., Ltd. Specific examples of the colloidal aluminum oxide include brands such as Alumina Sol 100 and Alumina Sol 520 available from Nissan Chemical Industries, Ltd.

When a metal alkoxide is used as a raw material for an oxide matrix, the oxide matrix is formed through hydrolysis of the metal alkoxide with a hydrophilic organic solvent. Examples of a metal alkoxide compound include: silicon alkoxysilane such as tetramethoxysilane, tetraethoxysilane, and tetrapropoxysilane; aluminum alkoxide such as aluminum ethoxide, aluminum isopropoxide, aluminum-n-butoxide, aluminum-sec-butoxide, and aluminum-tert-butoxide; titanium alkoxide such as tetramethoxy titanium, tetraethoxy titanium, tetra-n-propoxy titanium, tetraisopropoxy titanium, tetra-n-butoxy titanium, and tetraisobutoxy titanium; and zirconium alkoxide such as zirconium tetramethoxide, zirconium tetraethoxide, zirconium tetra-n-propoxide, zirconium tetraisopropoxide, zirconium tetra-n-butoxide, and zirconium tetra-t-butoxide. Examples of the organic solvent include: alcohols such as methanol, ethanol, butanol, ethylene glycol, and ethylene glycol-mono-n-propyl ether; and various ketones such as acetone, methyl ethyl ketone, methyl isobutyl ketone, and cyclohexanone. When a solution of the above-mentioned alkoxide compound is prepared, hydrolysis is performed by adding an acid catalyst or stabilizer which controls hydrolysis of an alkoxyl group or by adding water, as necessary. Examples of the catalyst include nitric acid, hydrochloric acid, sulfuric acid, phosphoric acid, acetic acid, and ammonia. Examples of the stabilizer include diketones such as acetylacetone and ethyl acetoacetate.

As a component that modifies the oxide matrix, organosiloxane having a basic functional group may be used. The term "basic functional group" refers to a functional group containing nitrogen which may form an acid-base structure with the phosphate group of the DNA. The siloxane having a basic functional group is obtained by hydrolyzing an alkoxysilane having the basic functional group. Specific examples of the alkoxysilane include the following compounds.

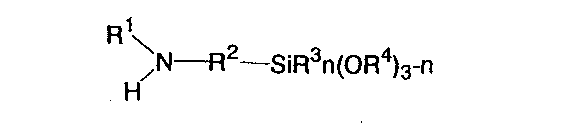

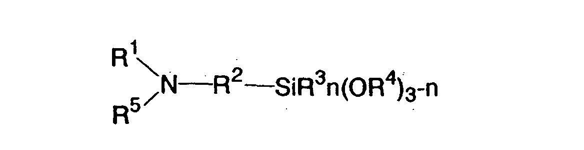

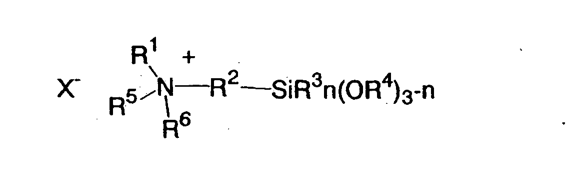

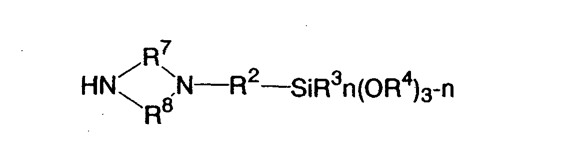

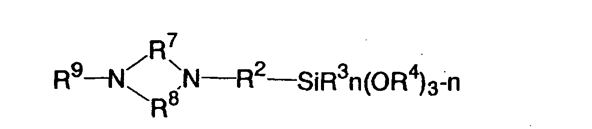

Here, in each formula, R¹ represents hydrogen or a monovalent hydrocarbon group having 1 to 8 carbon atoms; each of R³, R⁴, R⁵, R⁶, and R⁹ independently represents a monovalent hydrocarbon group having 1 to 8 carbon atoms; R⁷ and R⁸ each represent a divalent hydrocarbon group having 1 to 8 carbon atoms; and R² represents a divalent hydrocarbon group having 1 to 8 carbon atoms or a divalent group having -NH-.

Specific examples of the compounds include H₂NC₃H₆Si(OCH₃)₃, H₂NC₃H₆SiCH₃(OCH₃)₂, H₂NC₃H₆Si(OC₂H₅)₃, H₂NC₃H₆SiCH₃(OC₂H₅)₂, (CH₃)HNC₃H₆Si(OCH₃)₃, (CH₃)HNC₃H₆SiCH₃(OCH₃)₂, (CH₃)HNC₃H₆SiCH₃(OC₂H₅)₃, (CH₃)HNC₃H₆SiCH₃(OC₂H₅)₂, (CH₃)₂NC₃H₆Si(OCH₃)₃, (CH₃)₂NC₃H₆SiCH₃(OCH₃)₂, (CH₃)₂NC₃H₆Si(OC₂H₅)₃, (CH₃)₂NC₃H₆SiCH₃(OC₂H₅)₂, (C₂H₅)₂NC₃H₆Si(OCH₃)₃, (C₂H₅)₂NC₃H₆Si(OC₂H₅)₃, H₂NC₂H₄NHC₃H₆Si(OCH₃)₃, H₂NC₂H₄NHC₃H₆SiCH₃(OCH₃)₂, H₂NC₂H₄NHC₃H₆Si(OC₂H₅)₃, H₂NC₂H₄NHC₃H₆SiCH₃(OC₂H₅)₂, (CH₃)HNC₂H₄NHC₃H₆Si(OCH₃)₃, (CH₃)HNC₂H₄NHC₃H₆SiCH₃(OCH₃)₂, (CH₃)HNC₂H₄NHC₃H₆Si(OC₂H₅)₃, CH₃HNC₂H₄NHC₃H₆SiCH₃(OC₂H₅)₂, (CH₃)₂NC₂H₄NHC₃H₆Si(OCH₃)₃, (CH₃)₂NC₂H₄NHC₃H₆SiCH₃(OCH₃)₂, (CH₃)₂NC₂H₄NHC₃H₆Si(OC₂H₅)₃, (CH₃)₂NC₂H₄NHC₃H₆SiCH₃(OC₂H₅)₂, Cl⁻(CH₃)₃N⁺C₃H₆Si(OCH₃)₂, and Cl⁻(C₄H₉)₃N⁺C₃H₆Si(OCH₃)₃. At least one kind of them can be used.

When the basic functional group is cyclic, specific examples of the alkoxysilane include the following compounds.

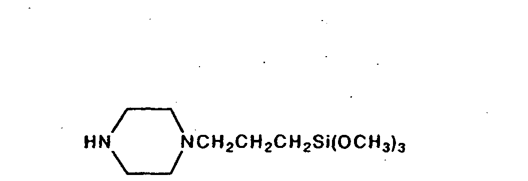

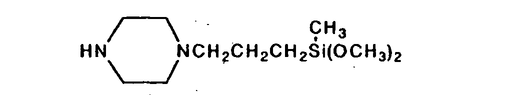

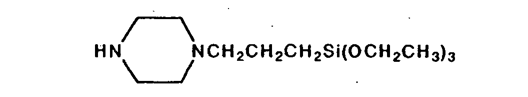

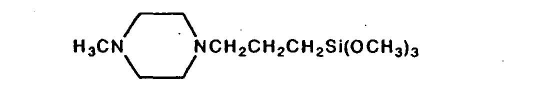

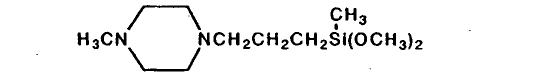

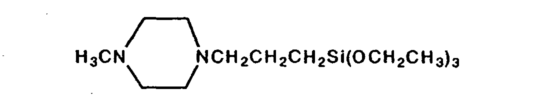

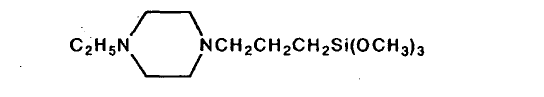

Examples of a method of hydrolyzing the above-mentioned alkoxysilane having a basic functional group includes: a method involving directly adding the alkoxysilane to water for hydrolysis; and a method involving adding necessary water to an organic dispersion medium such as alcohol or ketone in advance for hydrolysis. After the hydrolysis, the solvent is replaced by water, as necessary, to obtain an aqueous solution of siloxane having a basic functional group. Examples of a method of modifying the oxide matrix include: a method involving immersing the matrix formed from the above-mentioned oxide raw material in a solution of the organosiloxane having a basic functional group to modify the oxide matrix; and a method involving forming an oxide matrix directly from siloxane having a basic functional group and a dispersion containing the above-mentioned oxide component.

A method of carrying a DNA on the oxide matrix is not particularly limited, and examples thereof include: a method involving immobilizing the DNA from the aqueous DNA solution on a matrix that has been formed in advance; and a method involving dispersing the DNA in a dispersion having a component of the matrix and directly solidifying the resultant dispersion to form a porous DNA- carrying product. It is preferable that the content (based on mass) of the DNA in the porous DNA-carried product be 0.01% to 15%, more preferably 0.1% to 10%. If the content of the DNA is 0.01% or more, the efficiency of developing the properties ascribable to the DNA may be improved. On the other hand, if the content of the DNA is 15% or less, no economical problems are caused and pores will be formed easily in the porous DNA-carried product. This allows migration of water into the porous DNA-carried product faster, so that not only the properties of the DNA on a surface layer but also the properties of the DNA inside the pores can be quickly developed.

The above-mentioned immobilizing step may include methods such as heating, spray drying, and vacuum drying the dispersion medium. It is preferable that heat be given to the resultant porous DNA-carried product to an extent that the DNA of the porous DNA-carried product will not be decomposed. A heat treatment temperature for the porous DNA-carried product is preferably 200°C or less, more preferably 150°C or less.

Examples of the porous DNA-carried product may include coating films on surfaces of substrates such as plates, tubes, fibers, woven fabrics, and nonwoven fabrics, in addition to powder and bulk as necessary. Further, the above-mentioned porous DNA-carried product powder, and the plates, tubes, fibers, woven fabrics, nonwoven fabrics, and the like each coated with the porous DNA-carried product may be used to form modules thereof. For example, the porous DNA-carried product powder can be packed into a column.

When an organic polymer is used as a matrix, the kind of the polymer is not particularly limited so far as the DNA can be immobilized in the polymer and the function thereof can be retained. An anionic polymer that can crosslink with the DNA and a metal ion can preferably be used. Examples of the anionic organic polymer that can be used include: natural polymers of alginic acid; polymers of acrylic acid, methacrylic acid, acryl phosphoric ester; and copolymers of acrylic acid, methacrylic acid, or acryl phosphoric ester with another vinyl monomer. At least one of these may be used as necessary. Examples of the copolymerizable vinyl monomer include acrylates, methacrylates, acrylonitrile, methacrylonitrile, styrene, nucleus-substituted styrenes, alkyl vinyl ethers, alkyl vinyl esters, perfluoroalkyl vinyl ethers, perfluoroalkyl vinyl esters, maleic acid, maleic acid anhydride, fumaric acid, itaconic acid, maleimide, and phenylmaleimide. Of the vinyl monomers, those which can be used particularly preferably are methacrylates, acrylonitrile, styrenes, maleimide, and phenylmaleimide.

A polymerization reaction is performed through solution polymerization, or polymerization by irradiation of ultraviolet rays or electron beam. A solution polymerization method is preferably used.

In solution polymerization, a monomer is dissolved in a solvent. Then, the solution polymerization is performed by using a polymerization initiator including: an azo-based initiator such as 2,2-azobisisobutyronitrile, 2,2-azobis(2,4-dimethylvaleronitrile), dimethyl-2,2-azobis(2-methylpropionate), or dimethyl-2,2-azobisisobutyrate; or a peroxide-based initiator such as lauryl peroxide, benzoyl peroxide, or tert-butyl peroctoate.

As the dispersion medium for use in vinyl polymerization, basically any dispersion medium may be used so far as the raw materials are soluble therein. Examples of the solvent that can be used include water, alcohols such as methanol, ketones such as acetone, and ethers such as ethylene glycol monomethyl ether. Also, a mixed solvent of two or more kinds of them may be used.

It is preferable that the solvent of the reaction system be used in a ratio by mass of about 1.0 to about 20.0, more preferably 1.5 to 10, and the polymerization initiator be used in a ratio by mass of about 0.005 to about 0.05, more preferably about 0.01 with respect to the monomer raw material component of 1. If the amounts of the solvent and polymerization initiator used fall out of the above-mentioned preferable ranges, the polymer gels to become insoluble in various solvents, causing a problem of failure in film formation or the like, which is undesirable. Polymerization conditions are as follows. That is, while the mixed solution is stirred, polymerization is performed at a temperature of 40°C or more, more preferably 60°C or more and the boiling point of the solvent or less.

In the raw materials, a ratio of an acidic component of acrylic acid, methacrylic acid, and acryl phosphoric ester is 5 mass% or more, more preferably 10 mass% or more. When the acidic component is more than 5 mass%, crosslinking with the DNA is possible.

A method of crosslinking with the anionic polymer involves introducing a metal ion into a mixture of the DNA and the anionic polymer for crosslinking. Divalent metal ions or metal ions having a valency greater than two are used as the metal ion species. Examples of the metal ion species include Mg²⁺, Ca²⁺, Sr²⁺, Ba²⁺, Mn²⁺, Cu²⁺, Zn²⁺, Al³⁺, Fe³⁺, Y³⁺, Co³⁺, In³⁺, La³⁺, Ti⁴⁺, Zr⁴⁺, and Sn⁴⁺. The amount of the metal ion in the DNA complex is within the range of preferably 0.05% to 50%, more preferably 0.1% to 30% in terms of the mass of metal oxide. When the amount of the metal ion added is within the range of 0.05% to 50%, the water resistance of the complex of the DNA and the matrix base member is developed and the double helix of the DNA is advantageously retained.

To obtain a uniform complex, a step' of mixing the polymer having the acidic component and the DNA is provided. Thereafter, the resultant mixture is brought into contact with a solution containing the metal ion to perform crosslinking. An aqueous mixed solution of an aqueous solution of an anionic polymer, alkali salt of the anionic polymer, or ammonium salt of the anionic polymer with an alkali salt of the DNA is preferably obtained and crosslinked with an aqueous solution of a water-soluble metal salt.

Depending on the final form of the DNA complex, the aqueous solution of the metal salt is added to the aqueous mixed solution to obtain a precipitate containing the DNA or a gel-product of the DNA, followed by drying to obtain a bulk-product of the DNA complex. Also, a fiber of the DNA complex can be obtained by appropriately adjusting the viscosity of the aqueous DNA mixed solution for gelling the aqueous DNA mixed solution into a fiber. Similarly, a sheet of the DNA complex can be obtained by gelling the aqueous DNA mixed solution into a sheet. Further, a plate, a tube, a fiber, a woven fabric, or a nonwoven fabric with a crosslinked DNA complex film can be obtained by: coating the DNA mixed solution onto a surface of the substrate such as a plate, a tube, a fiber, a woven fabric, or a nonwoven fabric; pre-drying the DNA mixed solution to form a DNA film; and then bringing the DNA film into contact with a solution of a metal salt to cause penetration of the metal ion.

Further, the above-mentioned DNA complex, and the plates, tubes, fibers, woven fabrics, or nonwoven fabrics each coated with the DNA complex can be used to form modules thereof. For example, the DNA complex may be packed into a column to extract a special substance in a gas or liquid. Also, a DNA hybrid-carried fiber or woven fabric can be formed into a module as a filter to form a filter material for milk or mother's milk.

Also in the case of the DNA immobilized on the above-mentioned porous inorganic or organic polymer matrix, the double helix structure of the DNA is stabilized in the presence of a salt. In particular, in the case of the porous inorganic matrix, movement of the double helix structure of the DNA will not break the inorganic matrix to hinder the function of the carried product.

Note that after bringing the double helix DNA-holding phase into contact with a liquid containing a separation target substance, at least one of the double helix DNA-holding phase having the separation target substance held or concentrated therein and the liquid in which the separation target substance has reduced concentration or from which the separation target substance is removed is recovered from the system. The recovered DNA-holding phase or the liquid can be used for a predetermined application as necessary.

Hereinafter, the present invention will be described in more detail by examples and the like. Hereinafter, "%" is based on mass.

### (Synthesis Example 1)

40 g of N-2-(aminoethyl)-3-aminopropyltrimethoxysilane was dropped into 200 g of distilled water and hydrolyzed at room temperature for 3 days. The obtained oligomer solution was concentrated at 60°C using an evaporator. Thereafter, 95 g of distilled water was added to the concentrate to obtain about 180 g of a siloxane solution N1 having a basic functional group. The siloxane solution had a solid content of 15.1%.

### (Synthesis Example 2)

40 g of the following organosilicon compound was dropped into 200 g of distilled water and hydrolyzed at room temperature for 3 days. 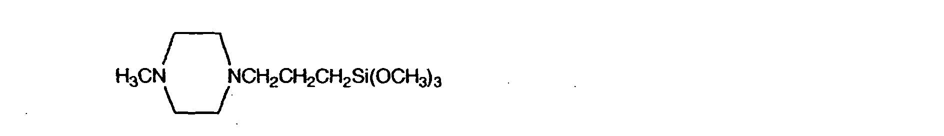 The resultant oligomer solution was concentrated at 60°C using an evaporator. Thereafter, 70 g of distilled water was added to the concentrate to obtain about 200 g of a siloxane solution N2 having a basic functional group. The siloxane solution had a solid content of about 14.7%.

### (Example 1)

A double-stranded DNA (molecular weight, 6 x 10⁶) obtained from 5 parts by weight of milt of a salmon was dissolved in 1,000 parts by weight of deionized water for 1 day to obtain an aqueous solution of DNA. 5 parts by weight of the siloxane solution N1 having a basic functional group was added to 100 parts by weight of 30% (by weight) silica sol (Nissan Chemical Industries, Ltd., SNOWTEX CM). The mixture was stirred for 30 minutes, and then 200 parts by weight of the aqueous solution of DNA was added thereto. Further, the resultant mixture was stirred slowly for 30 minutes, and then the dispersion medium was removed at 50°C using an evaporator. Thereafter, the resultant was dried at 60°C for 15 hours. The obtained agglomerate was pulverized, and particles having a size of 1 mm to 4 mm were taken out using a sieve to obtain a porous DNA-carried product 1 (having a DNA content of about 3.2% by weight).

An ethidiumbromide adsorption test was performed using the obtained porous DNA-carried product 1. 0.5 part by weight of the porous DNA-carried product was immersed in 50 parts by weight of 50 ppm ethidium bromide containing 100 ppm sodium chloride. After 3 hours, coloring due to ethidium bromide in a supernatant decreased and the porous DNA-carried product turned red. Upon irradiation of 366 nm ultraviolet rays, the porous DNA-carried product showed orange fluorescence, which indicated that an intercalation function of the DNA with toxic compounds each having a planar structure was retained. No cracks were observed in the porous DNA-carried product.

Further, measurement of a specific surface area of the porous DNA-carried product using a nitrogen adsorption method gave a specific surface area of 121 m²/g.

### (Example 2)

1 part by weight of the porous DNA-carried product of Example 1 was immersed in 50 parts by weight of 50 ppm ethidium bromide containing 200 ppm potassium chloride. After 3 hours, the coloring due to ethidium bromide in the supernatant decreased and the porous DNA-carried product turned red. Upon irradiation of 366 nm ultraviolet rays, the porous DNA-carried product showed orange fluorescence, which indicated that the intercalation function of the DNA with toxic compounds each having a planar structure was retained. No cracks were observed in the porous DNA-carried product.

### (Example 3)

4 parts by weight of the siloxane solution N2 having a basic functional group (Synthesis Example 2) was added to 100 parts by weight of 30% (by weight) silica sol (Nissan Chemical Industries, Ltd., SNOWTEX CM). The mixture was stirred for 30 minutes, and then 150 parts by weight of the aqueous solution of DNA (Example 1) was added thereto. Further, the resultant mixture was stirred slowly for 30 minutes and then the dispersion medium was removed at 50°C using an evaporator. Thereafter, the resultant was dried at 60°C for 15 hours. The obtained agglomerate was pulverized, and particles having a size of 1 mm to 4 mm were taken out using a sieve to obtain a porous DNA-carried product 2 (having a DNA content of about 2.4% by weight).

The ethidium bromide adsorption test was performed using the obtained porous DNA-carried product 2. 0.5 part by weight of the porous DNA-carried product was immersed in 50 parts by weight of 50 ppm ethidium bromide containing 100 ppm sodium chloride. After 3 hours, the coloring due to ethidium bromide in the supernatant decreased and the porous DNA-carried product turned red. Upon irradiation of 366 nm ultraviolet rays, the porous DNA-carried product showed orange fluorescence, which indicated that the intercalation function of the DNA with toxic compounds each having a planar structure was retained. No cracks were observed in the DNA-carried product.

1 part by weight of the obtained porous DNA-carried product was immersed in 50 parts by weight of an aqueous solution of 50 ppm sodium chloride. Even after 1 week, no cracks formed in the porous DNA-carried product.

### (Example 4)

3 parts by weight of the siloxane solution N2 having a basic functional group (Synthesis Example 2) was added to 100 parts by weight of 30% (by weight) silica sol (Nissan Chemical Industries, Ltd., SNOWTEX CM). The mixture was stirred for 30 minutes, and then 300 parts by weight of the aqueous solution of DNA (Example 1) was added thereto. Further, the resultant mixture was stirred slowly for 30 minutes, and then the dispersion medium was removed at 50°C using an evaporator. Thereafter, the resultant was dried at 60°C for 15 hours. The obtained agglomerate was pulverized, and particles having a size of 1 mm to 4 mm were taken out using a sieve to obtain a porous DNA-carried product 3 (having a DNA content of about 4.7% by weight).

The ethidium bromide adsorption test was performed using the obtained porous DNA-carried product 3. 0.5 part by weight of the porous DNA-carried product was immersed in 50 parts by weight of 50 ppm ethidium bromide containing 100 ppm sodium chloride. After 3 hours, the coloring due to ethidium bromide in the supernatant decreased and the porous DNA-carried product turned red. Upon irradiation of 366 nm ultraviolet rays, the porous DNA-carried product showed orange fluorescence, which indicated that the intercalation function of the DNA with toxic compounds each having a planar structure was retained. No cracks were observed in the DNA-carried product.

### (Example 5)

1 part by weight of the porous DNA-carried product 3 of Example 4 was immersed in 50 parts by weight of 50 ppm ethidium bromide containing 200 ppm potassium chloride. After 3 hours, the coloring due to ethidium bromide in the supernatant decreased and the porous DNA-carried product turned red. Upon irradiation of 366 nm ultraviolet rays, the porous DNA-carried product showed orange fluorescence, which indicated that the intercalation function of the DNA with toxic compounds each having a planar structure was retained. No cracks were observed in the DNA-carried product.

### (Example 6)

15 cc of 5 wt% NaCl and an aqueous 0.05% DNA solution (molecular weight of DNA: 6,000,000) were injected into a tube of a regenerated cellulose dialysis membrane having a diameter of about 2 cm and a molecular weight cutoff of 10,000, and both ends of the tube were sealed. The tube containing the DNA liquid was immersed in an aqueous solution of 50 ppm ethidium bromide containing 5 wt% NaCl that had been preliminarily heated at 65°C in an oil bath and stirred. Observation for 1 day revealed that the color of the DNA tube was considerably deeper than that of the surrounding ethidium bromide solution. Upon irradiation of 366 nm ultraviolet rays, intense orange fluorescence was observed. After cooling the tube, an absorbance of ethidium bromide was measured using a spectrophotometer, which indicated that the concentration of ethidium bromide was at least 150 ppm.

### (Comparative Example 1)

Durability of the porous DNA-carried product of Example 4 in highly pure water was examined. 0.1 part by weight of the porous DNA-carried product 2 was immersed in 100 parts by weight of distilled water. In a stage where bubbles were generated from the surface of the DNA-carried product, the DNA-carried product mostly retained its shape but partly cracked into powder of 0.1 mm to 1 mm. After 2 hours, no significant changes were observed. After 2 days, the concentration of DNA in the supernatant was measured using a spectrophotometer. The absorbance near 260 nm ascribable to DNA was about 0.03. Most of the DNA of the porous DNA-carried product was not eluted but the mechanical strength of the porous DNA-carried product was low.

### (Comparative Example 2)

An aqueous 0.05% DNA solution (molecular weight of DNA: 6,000,000) without 15 cc of a salt was injected into a tube of a regenerated cellulose dialysis membrane having a diameter of about 2 cm and a molecular weight cutoff of 10,000, and both ends of the tube were sealed. The tube containing the DNA liquid was immersed in an aqueous solution of 50 ppm ethidium bromide that had been preliminarily heated at 65°C in an oil bath and stirred. Observation for 1 day revealed that the color of the DNA tube substantially did not change as compared with that of the surrounding ethidium bromide solution. Upon irradiation of 366 nm ultraviolet rays, orange fluorescence of low intensity was observed. After cooling the tube, an absorbance of ethidium bromide was measured using a spectrophotometer, which indicated that the concentration of ethidium bromide was about 60 ppm. The adsorption performance was considerably weaker than that in the presence of a salt.

The present invention is not limited to the above embodiments and various changes and modifications can be made within the scope of the present invention. Therefore to apprise the public of the scope of the present invention, the following claims are made.

## Claims

1. A system for treating a liquid containing a separation target substance, comprising:
a treating region having arranged therein a double helix DNA-holding phase;
liquid supply means for supplying a liquid containing the separation target substance to the treating region; and
recover means for recovering at least one of 1) and 2) below,
1) the DNA-holding phase in which the separation target substance is held or concentrated through contact with the double helix DNA-holding phase, and
2) a liquid in which the separation target substance is removed or a concentration of the separation target substance is decreased,
wherein a concentration of a salt compound in a liquid phase where the contact of the double helix DNA with the separation target substance occurs is 0.02 mass% or more.

2. The treating system according to claim 1, wherein the salt compound contains at least one kind selected from the group consisting of sodium chloride, potassium chloride, calcium chloride, and magnesium chloride.

3. The treating system according to claim 1 or 2, wherein the salt compound has a concentration of 0.1 mass% or more.

4. The treating system according to claim 1, wherein the double helix DNA-holding phase comprises a solid phase, and the solid phase contains an inorganic porous base member.

5. The treating system according to claim 4, wherein the inorganic porous base member comprises porous silica.

6. The treating system according to claim 5, wherein the porous silica is one formed from at least colloidal silica and siloxane having a basic functional group.

7. The treating system according to claim 1, wherein the double helix DNA-holding phase comprises a solid phase, and the solid phase contains an organic polymer base member.

8. The treating system according to claim 7, wherein the organic polymer contains a vinyl polymer having an anionic functional group.

9. The treating system according to claim 8, wherein the anionic functional group comprises a phosphate acid functional group.

10. A separation treatment method for separating a separation target substance from a liquid using the treating system according to any one of claims 1 to 9, the method comprising the steps of:
providing a liquid containing the separation target substance;
supplying the liquid to the treating region and bringing the liquid into contact with the double helix DNA-holding phase at a salt compound concentration of 0.02 mass% or more to have the separation target substance in the liquid held by the double helix DNA-holding phase; and
recovering at least one of the DNA-holding phase in which the separation target substance is held or concentrated through contact of the liquid with the double helix DNA-holding phase, and the liquid from which the separation target substance is removed or in which the separation target substance has a decreased concentration.
